# EUROPÄISCHE PATENTSCHRIFT

(11) **EP 0 708 823 B1**
(45) Veröffentlichungstag und Bekanntmachung des Hinweises auf die Patenterteilung: **23.09.1998**
(21) Anmeldenummer: 94924241.6
(22) Anmeldetag: 06.07.1994
(51) Int. Cl.: C12N 5/00, C12M 3/06

(54) **VERFAHREN UND VORRICHTUNG ZUR BEHANDLUNG VON ZELLKULTUREN**
PROCESS AND DEVICE FOR TREATING CELL CULTURES
PROCEDE ET DISPOSITIF PERMETTANT DE TRAITER DES CULTURES CELLULAIRES

(30) Priorität: 08.07.1993 DE 4322746
(43) Veröffentlichungstag der Anmeldung: 01.05.1996
(73) Patentinhaber: BADER, Augustinus, D-31275 Lehrte (DE)
(72) Erfinder: BADER, Augustinus, D-31275 Lehrte (DE)
(74) Vertreter: Lorenz, Werner, Dipl.-Ing.
(86) Internationale Anmeldenummer: EP9402217
(87) Internationale Veröffentlichungsnummer: WO9502037

(56) Entgegenhaltungen:
- WO-A-89/01967
- WO-A-93/18133
- PATENT ABSTRACTS OF JAPAN vol. 14, no. 254 (C-0724) 31. Mai 1990 & JP,A,20 071 755 (TERUMO CORP) 12. März 1990
- ANNUAL INTERNATIONAL CONFERENCE OF THE IEEE ENGINEERING IN MEDECINE AND BIOLOGY SOCIETY, Bd.12, Nr.1, September 1990, US Seiten 443 - 444, XP000239414 SCOTT L. NYBERG ET AL. 'BILIRUBIN CONJUGATION IN A THREE COMPARTMENT HOLLOW FIBER BIOREACTOR'

## Beschreibung

Die Erfindung betrifft ein Verfahren zur Behandlung von Zellkulturen, insbesondere von Hepatozyten, auf Hohlfasern, die wenigstens teilweise gas- und/oder flüssigkeitsdurchlässig sind, wobei die Zellkulturen direkt oder indirekt über eine erste Matrixschicht auf oder in die Hohlfasern aufgebracht werden und Suspensionsmedium als Kulturmedium durch oder um die Hohlfasern geleitet wird und wobei die Zellen der Zellkulturen auf der von den Hohlfasern abgewandten Seite mit einer Matrix abgedeckt werden. Außerdem betrifft die Erfindung eine Vorrichtung zur Durchführung des Verfahrens.

In der Medizin und in der Pharmazie ist es häufig erforderlich, Versuche mit Zellkulturen, insbesondere mit Leberzellen (Hepatozyten), durchzuführen. Dies gilt z.B. zum Züchten, zu deren Beobachtung, insbesondere zur Beobachtung von Reaktionen auf Fremd- und/ oder Giftstoffen, zur Konservierung und dergleichen.

Darüber hinaus wird die Suche nach geeignetem Organersatz immer wichtiger.

Bei bekannten Systemen zur Kultur von Leberzellen in sogenannten Hohlfaserreaktoren wurden bisher die Zellen entweder innerhalb oder außerhalb der Hohlfasern durch Suspendierung in einer Lösung, wie z.B. einem Kulturmedium, in flüssiger Form eingefüllt. Nach der Adhäsion dieser Zellen auf den Fasern oder auch unter Belassung von nicht-adhärenten Zellen in den Zwischenräumen zwischen den adhärierten Zellen in Form einer Aggregatbildung, wurde das System für den jeweiligen Einsatzzweck verwendet.

Der entscheidende Nachteil der bekannten Verfahren ist, daß interzelluläre Kontakte zwischen den einzelnen Hepatozyten nur ungenügend oder zum Teil auch überhaupt nicht ausgebildet wurden.

Der Verlust von zellulärer Morphologie bringt nicht nur einen Funktionsverlust, sondern verhindert auch die Regenerationsfähigkeit nach einer Stimulierung der Zellen mit Wachstumsfaktoren in einem längerfristigen Kultursystem. Die längerfristige Kultivierung wäre jedoch von großer Bedeutung bei der Verwendung von humanen Hepatozyten, da diese nicht in beliebiger Menge verfügbar sind.

In "Annual International Conference of the IEEE Engineering in Medicine and Biology Society 12(1), 443-444, 1990", ist ein Verfahren zur Behandlung von Zellkulturen nach der eingangs erwähnten Art beschrieben. Dabei werden Zellen in einer dreidimensionalen Matrix eingelagert, wobei diese nach der Gel Entrapment-Methode zur gleichen Zeit mit dem Kollagen auf Hohlfasern aufgebracht werden. Dabei kommt es rasch zu einer Gelierung des Kollagens und damit zu einer dreidimensionalen Orientierung der Zellen und daraus resultierend einer unvollständigen Kontaktbildung der Zellen untereinander. Dies führt zu erheblichen Funktionsverlusten dieser Zellen und zwar bereits nach wenigen Tagen.

Der vorliegenden Erfindung liegt die Aufgabe zugrunde, ein Verfahren der eingangs erwähnten Art zu schaffen, mit dem eine Massenkultur möglich wird, wobei man so weit wie möglich einen "In vivo"-Zustand, insbesondere eine möglichst lange Lebensdauer erreichen möchte.

Erfindungsgemäß wird diese Aufgabe dadurch gelöst, daß die Matrix, welche die Zellen auf der von den Hohlfasern abgewandten Seite abdeckt, als eigene äußere Matrixschicht in gekühltem Zustand zur Überschichtung der Zellen erst aufgebracht wird, wenn eine strukturierte Form von adhärierten Zellen vorliegt.

Mit dem erfindungsgemäßen Verfahren werden die Zellkulturen in einer extra-zellulären Matrixschicht im Sinne einer an sich bekannten Sandwichtechnik eingebettet. Dabei kommt es zu einer Reorganisation der Zellform und einer erneuten Ausbildung von Mikrovilli auf der oder den den Matrixflächen zugewandten Seiten. Dies entspricht der natürlichen Form der Leberzellen und fördert den Stoffaustausch nach Art von sinusoidalen Membranen. Wesentlich neben diesen morphologischen Vorteilen ist jedoch auch der Funktionserhalt dieser Zellen.

Eines der wesentlichen Merkmale der Erfindung stellt damit die zweite, nämlich die äußere Matrixschicht dar, die die Zellkulturen umhüllt. Diese Umhüllung wird jedoch erst dann vorgenommen, wenn die Zellkulturen auf den Hohlfasern aufgebracht und dort adhäriert sind. Dies kann dabei direkt erfolgen oder über eine dazwischenliegende erste Matrixschicht. Die Verwendung einer ersten Matrixschicht hängt davon ab, welche Art von Zellkulturen behandelt werden sollen und welches Material für die Hohlfasern verwendet wird. Bestimmte Zellkulturen benötigen nämlich im Zusammenhang mit Hohlfasern keine gesonderte Matrixschicht als Zwischenlage, sondern können direkt auf den Oberflächen von bestimmten Hohlfasern adhärieren.

In einer vorteilhaften Ausgestaltung der Erfindung kann dabei vorgesehen sein, daß die Zellen auf einer Vielzahl von Hohlfasern jeweils auf dessen Außenseite aufgebracht werden, wobei jeweils die Zellen außenseitig von der Matrixschicht abgedeckt werden.

Je nach Durchmesser der Hohlfasern können die Zellkulturen und die Matrixschicht bzw. die Matrixschichten auch nach innen in die Hohlräume der Hohlfasern aufgebaut werden. Aus praktischen Gründen wird man jedoch im allgemeinen eher das Suspensions- bzw. Kulturmedium innen durch die Hohlfasern einleiten und nach außen zur Versorgung der Zellkulturen durch die Wände der Hohlfasern durchtreten lassen.

Um ein vorzeitiges Gelieren von Kollagen, das im allgemeinen als Hauptbestandteil in der Matrixschicht vorhanden ist, zu vermeiden, werden die Hohlfasern zum Aufbringen der ersten Matrixschicht auf Temperaturen von weniger als 15°C, vorzugsweise weniger als 4°C, abgekühlt.

Die Zellkulturen können in einer vorteilhaften Weise über einen Träger in Form eines Suspensionsmediums aufgebracht werden, wobei das Suspensionsmedium nach dem Auftragen der Zellkulturen auf die Hohlfasern abfließt.

Durch das Abfließen des Suspensionsmediums entstehen entsprechende Freistellen, in die dann beim Aufbringen der zweiten Matrixschicht diese einfließen kann. Auf diese Weise wird erreicht, daß die Zellen bipolar adhäriert bzw. bipolar aktiviert sind. Dadurch sind sie morphologisch in gleicher Weise wie in der Natur auch intakt und funktional stabiler.

In einer sehr vorteilhaften Weiterbildung der Erfindung kann vorgesehen sein, daß zur Bildung von dreidimensionalen Co-Kulturen mit der zu behandelnden Zellkultur nichtparenchymale Zellen auf der dem Kulturmediumzufluß zugewandten Seite aufgebracht werden.

Im allgemeinen wird man die nichtparenchymalen Zellen noch vor dem Eingießen der ersten Matrixschicht auf die Hohlfasern durch ein konventionelles Einbringen in flüssiger Suspension aufbringen.

Alternativ dazu können die nichtparenchymalen Zellen jedoch auch innerhalb der Hohlfasern an dessen Innenwänden aufgebracht werden, wodurch Interaktionen mit den sich außerhalb der Hohlfasern befindlichen bipolar adhärierten Zellen ebenfalls möglich sind. Die bipolar adhärierten Zellen, z.B. Hepatozyten, sind auf diese Weise von einem Schirm nichtparenchymaler Zellen außerhalb der Matrixschichten umgeben. Die nichtparenchymalen Zellen wirken praktisch als Barriere. Sie können bestimmte Substanzen ausfiltern, modifizieren und reagieren auch auf Substanzen, bevor diese Substanzen auf die Hepatozyten treffen.

Nachfolgend ist ein Ausführungsbeispiel der Erfindung anhand der Zeichnung prinzipmäßig dargestellt.

Es zeigt:

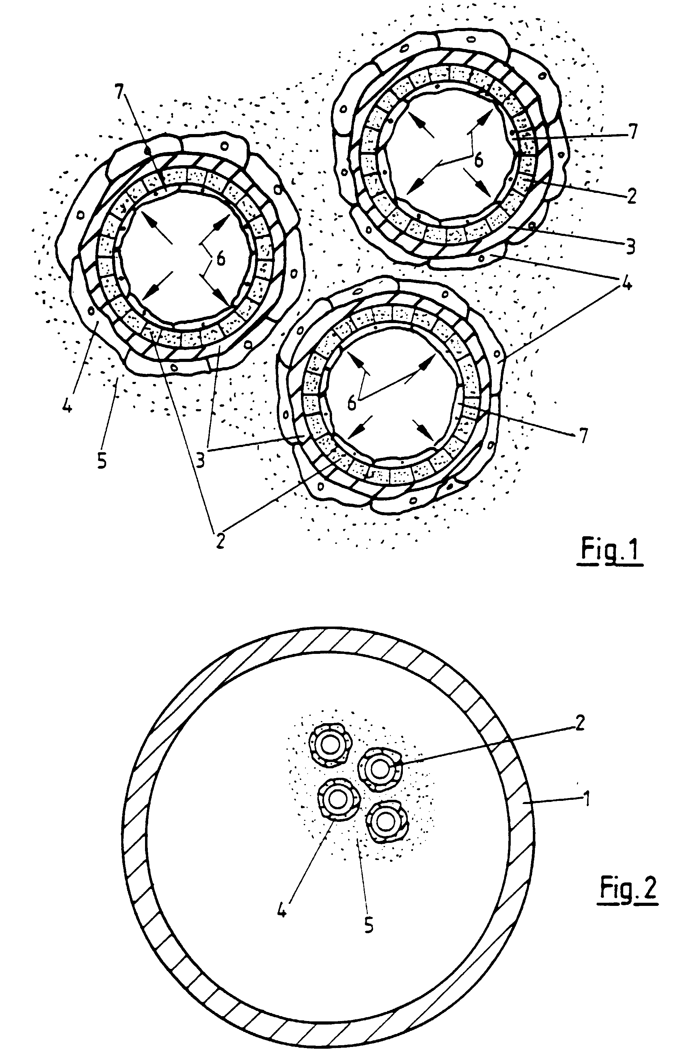

- Fig. 1: eine vergrößerte Darstellung von drei Hohlfasern mit Hepatozyten und Matrixschichten sowie mit nichtparenchymalen Zellen;
- Fig. 2: einen Querschnitt durch eine Vorrichtung in Form eines Reaktors mit darin angeordneten Hohlfasern (nur teilweise dargestellt).

In einem Hohlfaserreaktor, der als ein röhrenförmiger Reaktor bzw. Behälter 1 ausgebildet sein kann, sind eine Vielzahl von Hohlfasern 2 nebeneinander angeordnet. Die Hohlfasern können aus porösem Polypropylen oder Polysulfon bestehen. Selbstverständlich sind jedoch auch noch andere Materialien möglich. Wesentlich ist lediglich, daß ein Stoffaustausch aufgrund einer entsprechenden Porosität durch die Wände der Hohlfasern hindurch möglich ist. Die Hohlfasern und damit auch der Behälter selbst können eine wirksame Länge von 20 bis 25 cm haben, wobei die Länge jedoch ebenfalls nur beispielsweise genannt ist.

Auf diese Hohlfasern wird eine erste Matrixschicht 3 jeweils außenseitig aufgebracht. Hierzu werden die Hohlfasern 2 abgekühlt, und zwar zum Einbringen der Matrixschicht, die z.B. aus einem Kollagen des Typs 1 besteht, wobei auch Laminine, Proteine oder andere organische Substanzen beigemischt sein können. Dies erfolgt in flüssiger Form, wobei zur Vermeidung eines vorzeitigen Gelierens eine Abkühlung auf 4°C erfolgt.

Wird die erste Matrixschicht 3 außenseitig aufgebracht, so wird im allgemeinen ein Innendurchmesser der Hohlfasern von z.B. 0,1 mm ausreichen. Soll der Aufbau mit der zu behandelnden Zellkultur nach innen erfolgen, so wird man im allgemeinen größere Durchmesser, z.B. 1, 2, 3 mm oder mehr, benötigen.

Die erste Matrixschicht 3 dient als eine Beschichtungsschicht für die Hohlfasern, um eine Adhäsion der Zellen 4 der zu behandelnden Zellkultur zu unterstützen. Bei einer entsprechenden Materialauswahl für die Hohlfasern 2 und einer damit harmonisierenden Zellkultur kann gegebenenfalls die erste Matrixschicht 3 auch entfallen.

In einem zweiten Schritt werden die Zellen 4 der Zellkultur in Suspensionsform in den Reaktor 1 eingebracht. Beim Einbringen in den Raum außerhalb der Hohlfasern 2 kommt es zu einem Abfließen des Suspensionsmediums in das Innere der Hohlfasern 2, wobei die Zellen 4 in dem Raum ausserhalb der vorher mit der ersten Matrixschicht 3 beschichteten Hohlfasern 2 auf dieser Matrixschicht verbleiben. Der tatsächliche Raumbedarf der Zellen 4 reduziert sich somit um den Volumenanteil des abgeflossenen Suspensionsmediums.

Anschließend soll vorzugsweise mindestens ein bis zwei Stunden gewartet werden, um den Zellen 4 die Möglichkeit der Adhäsion auf der ersten Matrixschicht 3 und für eine Mikrovilliausbildung zu geben, damit ungehindert interzelluläre Kontakte geschaffen werden können. Dabei kommt es auch zur Ausbildung von Gallenkanälchen an den interzellulären Kontaktstellen im Bereich der apikalen Zellmembranseiten der Zellen 4. Als Zellen 4 werden bei dem Ausführungsbeispiel Hepatozyten verwendet.

In einem dritten Schritt wird eine getrennte zweite Matrixschicht 5 in den Reaktor eingebracht. Hierfür wird ein Matrixgemisch bei vorzugsweise 4°C in einer sauren Lösung mit einem basischen Mediumkonzentrat im Verhältnis 1 : 10 vermischt und anschließend in flüssiger Form in den Reaktor 1 eingefüllt. Bei dem Mediumkonzentrat handelt es sich um konzentriertes Kulturmedium, das durch die Konzentration geliert ist.

Das Einfüllen geschieht in die durch das Abfließen des Suspensionsmediums freiwerdenden Räumen, wobei auch im allgemeinen die Zwischenräume zwischen den Hohlfasern 2 außenseitig ausgefüllt werden. Praktisch bedeutet dies, daß man die zweite Matrixschicht 5 erst dann einbringt, wenn eine strukturierte Form der bereits adhärierten Zellen 4 vorliegt.

Durch die zweite Matrixschicht 5 werden die Zellen 4 außenseitig völlig umhüllt. Auf diese Weise können sie sich sowohl innenseitig als auch außenseitig, d.h. bipolar, verankern. Damit entsteht eine dreidimensionale Struktur, wobei die Zellen in gleicher Weise wie in der Natur interzelluläre Kontakte ausgebildet haben und in einer entsprechenden Wechselwirkung stehen.

Bei einer einsetzenden Erwärmung und einer pH-Änderung kommt es nach dem Einfüllen der zweiten Matrixschicht 5 zu einer Verfestigung dieser Matrixschicht, wobei sich ein inniger Kontakt mit den Zellmembranoberflächen bildet. Durch die Beschichtung und die Umhüllung können die Zellen 4 auf diese Weise auch in einem massenkulturfähigen System mit der Vielzahl von Hohlfasern 2 Mikrovilli auf der der zweiten, äußeren Matrixschicht 5 zugewandten Zellmembranseite, der sinusoidalen Seite, ausbilden.

Würde diese zweite Matrixschicht 5 nicht vorhanden sein, würde der daraus resultierende morphologische Nachteil zu einem kontinuierlichen Funktionsverlust der Zellen führen. Die Möglichkeit einer Wanderung und Ausbreitung der Zellen 4 innerhalb der beiden Matrixschichten, wie z.B. auf Proliferationsreize, ist möglich.

Zur Ausnutzung der Proliferations- und Regenerationsfähigkeit der erfindungsgemäß bipolar adhärierten Hepatozyten werden Wachstumsfaktoren vorgesehen.

Kulturmedium, wie z.B. Blut, Plasma, Proteine und/oder ein sauerstoffhaltiges Nährmedium zur Versorgung der Zellen 4, wird von einer Stirnseite des zylinderförmigen Reaktors 1 aus in das Innere der Hohlfasern 2 eingebracht, wobei es in Richtung der Pfeile 6 durch die Wände der Hohlfasern 2 von innen nach außen aufgrund deren porösen Charakters hindurchtritt. In gleicher Weise ist auch ein Austausch von Substanzen von außen nach innen möglich. Auf der anderen Stirnseite des Behälters 1 fließt restliches Kulturmedium wieder ab.

Um dreidimensionale Co-Kulturen zu schaffen, können nichtparenchymale Zellen 7.jeweils auf die Innenwände der Hohlfasern 2 aufgebracht werden. Statt einer Aufbringung auf der Innenseite der Hohlfasern können diese auch aussenseitig auf die Hohlfasern 2 aufgebracht werden, wenn diese noch vor der ersten Matrixschicht 3 eingegossen werden. Aufgrund der Porosität der Hohlfasern 2 können jedoch auch bei einer Anordnung auf den Innenseiten der Hohlfasern 2 Interaktionen mit den sich außerhalb der Hohlfasern 2 befindlichen Zellen 4 stattfinden.

Als nichtparenchymale Zellen 7 können z.B. Endothelzellen, Kupfferzellen, Itozellen und Pitzellen vorgesehen werden.

## Patentansprüche

1. Verfahren zur Behandlung von Zellkulturen, insbesondere von Hepatozyten, auf Hohlfasern (2), die wenigstens teilweise gas- und/oder flüssigkeitsdurchlässig sind, wobei die Zellkulturen direkt oder indirekt über eine erste Matrixschicht (3) auf oder in die Hohlfasern (2) aufgebracht werden und Suspensionsmedium als Kulturmedium durch oder um die Hohlfasern (2) geleitet wird und wobei die Zellen (4) der Zellkulturen auf der von den Hohlfasern (2) abgewandten Seite mit einer Matrix abgedeckt werden, dadurch gekennzeichnet, daß die Matrix, welche die Zellen (4) auf der von den Hohlfasern (2) abgewandten Seite abdeckt, als separate äußere Matrixschicht (5) in gekühltem Zustand zur Überschichtung der Zellen (4) erst aufgebracht wird, wenn eine strukturierte Form von adhärierten Zellen (4) vorliegt.

2. Verfahren nach Anspruch 1, dadurch gekennzeichnet, daß die Zellen (4) auf einer Vielzahl von Hohlfasern (2) jeweils auf dessen Außenseite aufgebracht werden, wobei jeweils die Zellen (4) außenseitig von der Matrixschicht (5) abgedeckt werden.

3. Verfahren nach Anspruch 2, dadurch gekennzeichnet, dass die Hohlfasern (2) mit einer ersten Matrixschicht (3) beschichtet werden, auf bzw. in die die Zellen (4) eingebracht werden, wonach außenseitig die zweite Matrixschicht (5) folgt.

4. Verfahren nach Anspruch 3, dadurch gekennzeichnet, dass zum Aufbringen der ersten Matrixschicht (3) auf die Hohlfasern (2) diese auf Temperaturen von weniger als 15°C abgekühlt werden.

5. Verfahren nach einem der Ansprüche 1 bis 4, dadurch gekennzeichnet, dass zum Aufbringen der Zellen (4) als Träger ein Suspensionsmedium verwendet wird, das nach dem Auftragen der Zellen (4) in die Hohlfasern (2) abfließt.

6. Verfahren nach einem der Ansprüche 1 bis 5, dadurch gekennzeichnet, dass die zweite Matrixschicht (5) in flüssiger Form in saurer Lösung mit einem basischen Mediumkonzentrat aufgebracht wird.

7. Verfahren nach einem der Ansprüche 1 bis 6, dadurch gekennzeichnet, dass zur Bildung von dreidimensionalen Co-Kulturen mit Zellen (4) nichtparenchymale Zellen (7) auf der dem Kulturmediumzufluß zugewandten Seite aufgebracht werden.

8. Verfahren nach Anspruch 7, dadurch gekennzeichnet, dass die nichtparenchymalen Zellen (7) innenseitig auf die Wände der Hohlfasern (2) aufgebracht werden.

9. Verfahren nach einem der Ansprüche 1 bis 8, dadurch gekennzeichnet, dass die Matrixschichten (3,5) Kollagen aufweisen.

10. Vorrichtung zur Durchführung des Verfahrens nach einem der Ansprüche 1 bis 9, dadurch gekennzeichnet, dass auf porösen Hohlfasern (2) Zellen (4) aufgebracht sind, die auf der von dem Kulturmedium abgewandten Seite von einer Matrixschicht (5) umgeben sind, wobei eine Vielzahl von Hohlfasern (2) in einem Behälter (1) angeordnet sind und wobei in die Hohlfasern (2) Zuleitungen für Kulturmedium münden, daß die Zellen (4) der Zellkulturen jeweils außen auf den Hohlfasern (2) angeordnet sind und daß die äußere Matrixschicht (5) in dem Behälter (1) den Raum zwischen den Hohlfasern (2) wenigstens teilweise ausfüllt.

## Claims

1. A process for treating cell cultures, in particular hepatocytes, on hollow fibres (2) which are at least partially gas and/or liquid permeable, whereby the cell cultures are applied directly or indirectly over a first matrix layer (3) on or into the hollow fibres (2) and suspension medium is conveyed as culture medium through or around the hollow fibres (2) and whereby the cells (4) of the cell cultures are covered by a matrix on the side faced away from the hollow fibres (2),
**characterised in that** the matrix which covers the cells (4) on the side faced away from the hollow fibres (2) is only applied as a separate outer matrix layer (5) in cooled state to coat the cells (4) if there is a structured form of adhered cells (4).

2. A process according to Claim 1,
**characterised in that** the cells (4) are applied to a plurality of hollow fibres (2) on their outer side in each case, whereby the cells (4) are covered on the outside by the matrix layer (5) in each case.

3. A process according to Claim 2,
**characterised in that** the hollow fibres (2) are coated with a first matrix layer (3), onto or respectively into which the cells (4) are introduced, after which the second layer (5) follows on the outside.

4. A process according to Claim 3,
**characterised in that** to apply the first matrix layer (3) onto the hollow fibres (2), said hollow fibres are cooled to temperatures of less than 15°.

5. A process according to one of Claims 1 to 4,
**characterised in that** to apply the cells (4) a suspension medium, which after the application of the cells (4) flows away into the hollow fibres (2), is used as a substrate.

6. A process according to one of Claims 1 to 5,
**characterised in that** the second matrix layer (5) is applied in liquid form in acid solution with a basic medium concentrate.

7. A process according to one of Claims 1 to 6,
**characterised in that** to form three-dimensional cocultures with cells (4), non-parenchyma cells (7) are applied to the side facing the culture medium inflow.

8. A process according to Claim 7,
**characterised in that** the non-parenchyma cells (7) are applied to the inside of the walls of the hollow fibres (2).

9. A process according to one of Claims 1 to 8,
**characterised in that** the matrix layers (3, 5) comprise collagen.

10. A device for performing the process according to one of Claims 1 to 9,
**characterised in that** cells (4), which are surrounded on the side remote from the culture medium by a matrix layer (5), are applied to porous hollow fibres (2), with a plurality of hollow fibres (2) being disposed in a receptacle (1) and with supply lines for culture medium opening into the hollow fibres (2),
**in that** the cells (4) of the cell cultures are disposed on the outside of the hollow fibres (2) in each case,
**and in that** the outer matrix layer (5) at least partially fills the space between the hollow fibres (2) in the receptacle (1).

## Revendications

1. Procédé de traitement de cultures cellulaires, en particulier d'hépatocytes, sur des fibres creuses (2) qui sont, au moins partiellement perméables aux gaz et/ou aux liquides, dans lequel les cultures cellulaires sont déposées directement ou indirectement sur une première couche de matrice (3) sur ou dans les fibres creuses (2) et le milieu de suspension étant conduit en tant que milieu de culture à travers ou autour des fibres creuses (2) et dans lequel les cellules (4) des cultures cellulaires sont recouvertes par une matrice du côté opposé aux fibres creuses (2), caractérisé en ce que la matrice, qui recouvre les cellules (4) du côté opposé aux fibres creuses (2), est d'abord déposée à l'état réfrigéré sous la forme d'une couche de matrice extérieure séparée (5) pour recouvrir les cellules (4), lorsqu'il existe une forme structurée de cellules adhérentes (4).

2. Procédé selon la revendication 1, caractérisé en ce que les cellules (4) sont déposées sur une pluralité de fibres creuses (2) sur leur côté extérieur, dans lequel les cellules (4) sont recouvertes, du côté extérieur, par la couche de matrice (5).

3. Procédé selon la revendication 2, caractérisé en ce que les fibres creuses (2) sont revêtues avec une première couche de matrice (3), sur laquelle respectivement dans laquelle les cellules sont (4) déposées, après quoi la deuxième couche de matrice (5) est déposée à l'extérieur.

4. Procédé selon la revendication 3, caractérisé en ce que pour déposer la première couche de matrice (3) sur les fibres creuses (2), celle-ci est refroidie à des températures inférieures à 15 °C.

5. Procédé selon l'une quelconque des revendications 1 à 4, caractérisé en ce qu'un milieu de suspension qui s'écoule dans les fibres creuses (2) après le dépôt des cellules (4) est utilisé comme support pour déposer les cellules (4).

6. Procédé selon l'une quelconque des revendications 1 à 5, caractérisé en ce que la deuxième couche de matrice (5) est déposée sous forme liquide en solution acide avec un concentré de milieu basique.

7. Procédé selon l'une quelconque des revendications 1 à 6, caractérisé en ce que, pour la formation de co-cultures tridimensionnelles avec les cellules (4), des cellules non-parenchymales (7) sont déposées du côté du flux de milieu de culture.

8. Procédé selon la revendication 7, caractérisé en ce que les cellules non-parenchymales (7) sont déposées à l'intérieur, sur la paroi des fibres creuses (2).

9. Procédé selon l'une quelconque des revendications 1 à 8, caractérisé en ce que les couches de matrice (3, 5) comportent du collagène.

10. Dispositif pour la mise en oeuvre du procédé selon l'une quelconque des revendications 1 à 9, caractérisé en ce que des cellules (4) sont déposées sur des fibres creuses poreuses (2), qui sont entourées d'une couche de matrice (5) du côté opposé au milieu de culture, dans lequel une pluralité de fibres creuses (2) sont disposées dans un conteneur (1) et dans lequel des conduits pour le milieu de culture aboutissent dans les fibres creuses (2), en ce que les cellules (4) des cultures cellulaires sont organisées à l'extérieur des fibres creuses (2) et en ce que la couche de matrice extérieure (5) remplit au moins partiellement l'espace entre les fibres creuses (2) dans le conteneur (1).
